# EUROPEAN PATENT APPLICATION

(11) **EP 1 363 119 A1**
(43) Date of publication of application: **19.11.2003**
(21) Application number: 02791980.2
(22) Date of filing: 25.12.2002
(51) Int. Cl.: G01N 21/27

(54) **INFORMATION MEASURING DEVICE**

(30) Priority: 26.12.2001 JP 2001393423; 08.10.2002 JP 2002294495
(71) Applicant: MATSUSHITA ELECTRIC INDUSTRIAL CO., LTD., Kadoma-shi, Osaka 571-8501 (JP)
(72) Inventor: UCHIDA, Shinji, Neyagawa-shi, Osaka 572-0807 (JP); OOSHIMA, Kiyoko, Shijonawate-shi, Osaka 575-0024 (JP); SHIOI, Masahiko, Katano-shi, Osaka 576-0021 (JP)
(74) Representative: Grünecker, Kinkeldey, Stockmair & Schwanhäusser Anwaltssozietät
(86) International application number: JP0213489
(87) International publication number: WO03056306

(57) **Abstract**

To provide an information measuring apparatus which enables stable and easy measurement of concentration information etc. of specific components in a sample even when fluids intervene between its optical device and the sample.

An information measuring apparatus including: a light source 1 ; an ATR device 2 which reflects incident light from the light source 1 onto the sample kept in contact with itself, receives the light returned from the sample, and reflects the received light to the outside; and light detection means 5 which detects the amount of the light reflected from the ATR device 2 to the outside, the ATR device 2 having a first area, part of its surface, where it is in contact with the sample and the light for irradiating the sample is input and a second area adjacent to the first area, the contact angle in the first area being larger than that of the second area, wherein the information in the sample is measured based on the signal obtained through the light detection means 5.

## Description

### BACKGROUND OF THE INVENTION

### Technical Field

The present invention relates to an information measuring apparatus such as concentration measuring apparatus of optically measuring the concentration information of specific components in a sample, etc. such as blood sugar level, water content, cholesterol, etc. of organisms by measuring returned light from the sample .

### Background Art

There have been proposed various methods of measuring specific components in a sample, particularly in an organism or solution, using an optical measuring apparatus.

For example, a method has been proposed of measuring a blood sugar level while closely touching upper and lower lips to a transparent attenuation total reflection (hereinafter referred to as ATR) device equipped with a pair of opposite reflection surfaces in parallel with each other. According to this method, an ATR prism, as an ATR device, made up of zinc selenide, silicon, germanium, etc. is held in a person's mouth, then light is made to enter the ATR device while the device is pressed with the upper and lower lips of the mouth, and total reflection is repeated at the interface of the reflection surfaces and the lips, to analyze the output light outside the ATR device (refer to, for example, Japanese Patent Laid-Open No. 9-113439).

Further, there has been proposed a method of determining a blood sugar level or blood ethanol concentration in which an ATR device consisting of ZnSe optical crystal and the like, is closely touched to the mucous membrane of person's lips, a laser light of wavelength of 9 to 11 micrometer is made to enter the ATR device and multiply reflect inside the same, and the attenuation total reflected light and scattering reflected light, etc. are analyzed (refer to, for example, Hideo Fukushima et al., "Noninvasive Method of Determining a Blood sugar level - Development of Optical Glucose Sensor -" BME, Japanese Society for Medical and Biological Engineering, Vol. 5, No. 8, 1991, 16-21).

According to these methods, concentrations of specific components, such as glucose concentration and cholesterol concentration, can be determined in real time and noninvasively. These methods are to apply evanescent light (known as penetrating light) to quantitative analyses. A slight amount of light traveling the inside of an ATR device enters the person's lips when total reflection is repeated at the interface of the reflection surfaces of the ATR device and the lips and is affected by the components of the body fluid existing in the lips. For example, in glucose, since the peaks of light absorption exist at wave numbers of 1033, 1080 cm⁻¹, when an organism is irradiated with light of such wave numbers, the amount of light absorption varies depending on the glucose concentration in the organism. Thus, the concentration information for components of body fluid can be obtained by measuring the light returning from the organism. The term "concentration information" herein used means the absolute value of concentration, the change of concentration with time, etc.

The entire disclosure of the above-described Japanese Patent Laid-Open No. 9-113439 and "Noninvasive Method of Determining a Blood sugar level - Development of Optical Glucose Sensor -" is incorporated herein by reference in its entirety.

However, the conventional optical measuring apparatuses described above have a problem in that in measuring the concentration information of a sample using an optical device, if fluid such as saliva intervenes between the optical device and the sample, variation is created in measured results because of the large change in the amount of signals resulting from the change in amount of the light reaching the sample with thickness of the fluid.

Generally, the depth to which evanescent light enters the object of measurement is of the order of its wavelength. Therefore, the evanescent light output from the optical device and allowed to enter the surface of an organism penetrates the superficial tissue over a distance of the order of its wavelength from the surface of the organism and then returns to the optical device. The above-described problem is significant in optical measuring apparatuses using an ATR device.

### Disclosure of the Invention

In light of the above-described problem, the object of the present invention is to provide an information measuring apparatus which enables stable and easy measurement of information, such as concentrations of specific components in a sample, even when fluids such as water, saliva and sweat intervenes between the optical device and the sample.

A first invention of the present invention is an information measuring apparatus of measuring information in a sample, comprising: a light source (1) ; an optical device (2) which irradiates light input from the light source (1) to the sample in contact with the optical device, receives the light returned from the sample, and outputs the received light; and light detection means (5) of detecting the amount of the light output from the optical device (2),
the optical device (2) having a first area (7) that is a part of the face in contact with the sample and to which the light for irradiating the sample inputs and a second area (13) adjacent to the first area (7), and the contact angle in the first area (7) being larger than that in the second area (13),
wherein the information in the sample is measured based on the signal obtained through the light detection means (5).

A second invention of the present invention is the information measuring apparatus according to the first invention of the present invention, wherein the information is the concentration information of specific components in the sample.

A third invention of the present invention is the information measuring apparatus according to the first invention of the present invention, wherein the first area (7) is formed of the material which the optical device (2) is formed of.

A fourth invention of the present invention is the information measuring apparatus according to the first invention of the present invention, wherein the second area (13) is formed of the material which the optical device (2) is formed of.

A fifth invention of the present invention is the information measuring apparatus according to the first invention of the present invention, wherein an concave portion is formed in part of the first area (7) or at least in part of the second area (13).

A sixth invention of the present invention is the information measuring apparatus according to the first or the third invention of the present invention, wherein the first area (7) is water repellent.

A seventh invention of the present invention is the information measuring apparatus according to the first or the fourth invention of the present invention, wherein the second area (13) is hydrophilic.

An eighth invention of the present invention is the information measuring apparatus according to the first invention of the present invention, wherein the first area (7) is water repellent and the second area (13) is hydrophilic.

A ninth invention of the present invention is the information measuring apparatus according to the first invention of the present invention, wherein the optical device (2) is an attenuation total reflection device

A tenth invention of the present invention is the information measuring apparatus according to. the fifth invention of the present invention, wherein the concave portion is connected to the surface (16) which is in contact with the sample and not on the optical path of the light passing through the optical device (2) and flow paths (3) are formed for discharging fluids intervening between the first area (7) and the sample.

An eleventh invention of the present invention is the information measuring apparatus according to the tenth invention of the present invention, wherein at least part of the flow paths (3) is hydrophilic.

A twelfth invention of the present invention is the information measuring apparatus according to the tenth invention of the present invention, wherein the flow paths (3) are formed so that they open in part of the first area (7).

A thirteenth invention of the present invention is the information measuring apparatus according to the fifth invention of the present invention, wherein the concave portion is formed in such a position and in such a size that it does not substantially prevent the optical path of the light passing through the optical device (2).

A fourteenth invention of the present invention is the information measuring apparatus according to the first invention of the present invention, wherein at least part of the surfaces (16) of the optical device (2), which are adjacent to the surface in contact with the sample and are not on the optical path of the light passing through the optical device (2), is hydrophilic.

A fifteenth invention of the present invention is the information measuring apparatus according to the first invention of the present invention, wherein at least one of the surfaces of the optical device (2) which are adjacent to the surface in contact with the sample is water repellent.

A sixteenth invention of the present invention is the information measuring apparatus according to the first invention of the present invention, further comprising fluid inflow preventive means (32) of preventing the fluids intervening between the first area (7) and the sample from flowing in the surface (11) of the optical device (2) through which the light from the light source (1) enters and the surface (12) of the optical device (2) through which the light returned from the sample is output into the light detection means (5) from the first or the second area (7 or 13) of the optical device (2).

A seventeenth invention of the present invention is the information measuring apparatus according to the first invention of the present invention, further comprising suction means (31) of sucking the fluids, which is provided on the surface of the optical device (2) adjacent to the surface in contact with the sample.

Then the invention made by the present inventors will be described.

A first invention is an information measuring apparatus of measuring information in a sample, including: a light source (1); an optical device (2) which irradiates incident light from the light source (1) onto the sample kept in contact with itself, receives the light returned from the sample, and outputs the received light to the outside; and light detection means (5) of detecting the amount of the light output from the optical device (2) to the outside,
the optical device (2) being water repellent at least in the portion where it is in contact with the sample and the light for irradiating the sample enters the portion,
wherein the information in the sample is measured based on the information obtained through the light detection means (5).

The above-described construction provides an information measuring apparatus which enables the solution of the above-described problem.

A second invention is an information measuring apparatus of measuring information in a sample, including: a light source (1) ; an optical device (2) which irradiates incident light from the light source (1) onto the sample kept in contact with itself, receives the light returned from the sample, and outputs the received light to the outside; and light detection means (5) of detecting the amount of the light output from the optical device (2) to the outside,
the optical device (2) having flow paths (3), which connect at least part of the portion of the optical device (2) in contact with the sample or part of the portion adjacent to part of the portion of the optical device (2) in contact with the sample with the surfaces (16) of the optical device (2) adjacent to the surface of the same in contact with the sample and not on the optical path of the light passing through the optical device (2) and discharge fluids intervening between the sample and the optical device (2) from the portion of the optical device (2) in contact with the sample,
wherein the information in the sample is measured based on the information obtained through the light detection means (5).

The above-described construction provides an information measuring apparatus which enables the solution of the above-described problem.

A third invention is an optical device (2) which irradiates incident light from a light source (1) onto a sample kept in contact with itself, receives the light returned from the sample, and outputs the received light to the outside,
wherein the optical device (2) measures information in the sample in such a manner that light detection means (5) detects the amount of light output to the outside and has a first area (7) that is at least a part of the portion in contact with the sample, where the light for irradiating the sample enters and a second area (13) adjacent to the first area (7), the contact angle of the first area being larger than that of the second area.

The above-described construction provides an optical device used in an information measuring apparatus which enables the solution of the above-described problem.

A fourth invention is an information measuring method of measuring information in a sample which uses an information measuring apparatus including: a light source (1) ; an optical device (2) which reflects incident light from the light source (1) onto the sample kept in contact with itself, receives the light returned from the sample, and reflects the received light to the outside; and light detection means (5) of detecting the amount of the light reflected from the optical device (2) to the outside,
the optical device (2) having a first area (7) that is at least a part of the portion in contact with the sample, where the light for irradiating the sample enters and a second area (13) adjacent to the first area (7) and the contact angle of the first area being larger than that of the second area,
wherein the information in the sample is measured based on the signal obtained through the light detection means (5).

The above-described construction provides an information measuring method which enables the solution of the above-described problem.

### BRIEF DESCRIPTION OF THE DRAWINGS

FIG. 1 is a schematic diagram showing a concentration measuring apparatus of measuring specific components as an example embodying the present invention;
FIG. 2 is a characteristic diagram showing the measurements of absorbance obtained using the concentration measuring apparatuses of measuring specific components of the above example and a comparative example;
FIG. 3 is a schematic diagram of a concentration measuring apparatus of measuring specific components as a modified example embodying the present invention; and
FIG. 4 is a schematic diagram of the concentration measuring apparatus as another modified example embodying the present invention.

### [Reference Numerals]

- 1: light source
- 2: ATR device
- 3: flow path
- 4: inclined plane
- 5: light detection means
- 6: top surface
- 7: water repellent provided portion
- 11: light-entering surface
- 12: light-reflecting surface
- 13: hydrophilicity provided portion
- 14: sample contacting area
- 31: suction means
- 32: step

### BEST MODE FOR CARRYING OUT THE INVENTION

In the following the embodiments of the present invention will be described in detail with reference to the accompanying drawings.

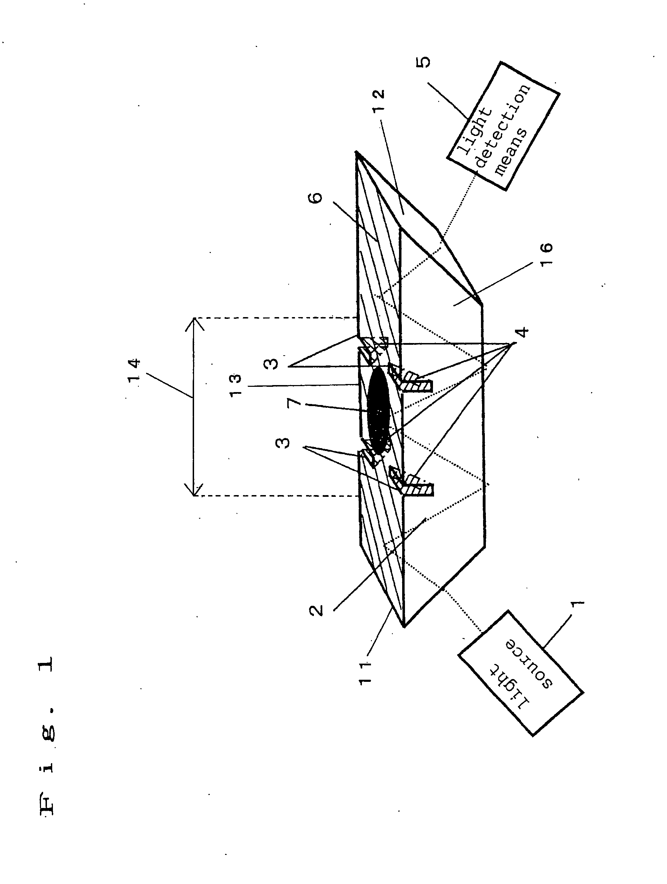

FIG. 1 is a schematic diagram showing a concentration measuring apparatus of measuring specific components as an example of the information measuring apparatuses embodying the present invention.

The concentration measuring apparatus according to this embodiment of measuring specific components is made up of a light source 1, an ATR device 2 as an example of the optical devices of the present invention, light detection means 5, and spectroscopic means (not shown) positioned between the light source 1 and the ATR device 2.

As a material for the ATR device 2, any materials known in the art can be used without any limitation. The materials include, for example, Si, Ge, SiC, diamond, ZnSe, ZnS and KrS. When measuring the concentration of a substance, such as glucose, whose absorption peaks appear in the infrared region of wave number 1033 and 1080 cm⁻¹, it is preferable from the viewpoint of high transmittance at infrared wavelength about 9 to 10 microns that the material is silicon or germanium having a low content of impurities, such as boron and phosphorus, and a resistivity of 100 Ωcm or more, more preferably 1500 Ωcm or more.

In the ATR device 2, in the middle of its top face 6, which is to come in contact with a sample, a water repellent provided portion 7 as an example of the first areas of the present invention is formed. The water repellent provided portion 7 can be formed by the methods known in the art and capable of providing high water repellent without special limitation. The methods include, for example, methods which use a fluorine resin, a silicon resin and a silicone resin, respectively, and a method which finely coarsens the water repellent provided surface.

When coating the top surface 6 with fluorine resin or silicon resin, preferably the thickness of the coating is 1 micron or less, taking into account the effects of these materials themselves on the measurements, and the coating can be a minimum thickness of one molecular layer to obtain sufficient water repellent effects.

Around the water repellent provided portion 7 of the top surface 6, a hydrophilicity provided portion 13 as an example of the second areas of the present invention is formed adjacently to the water repellent provided portion 7. The hydrophilicity provided portion 13 can be formed by the methods known in the art without any limitation. The methods include, for example, a method which uses a surfactant, methods which form films of zinc plating, aluminium fluoride, water glass, colloidal silica, alumina, and a high polymer resin, respectively, a method which forms a mixed film of silica-based material and a high polymer resin, and a method which use a phenomenon that titanium oxide is made hydrophilic by optical excitation. When forming the hydrophilicity provided portion 13 adjacently to the water repellent provided portion 7, it is preferable that the thickness of the hydrophilic material is 1 micron or less so as to reduce its effect on the optical properties of the ATR device 2.

Around the water repellent provided portion 7 are formed four flow paths 3, one of the openings of each flow path is in communication with one part of the hydrophilicity provided portion 13 and the other is in communication with a side surface 16. Inside each flow path 3 is formed an inclined plane 4 which is provided with hydrophilicity. These flow paths 3 are formed at such a position and in such size that do not interrupt the passage of the light irradiated from the light source 1. The area corresponding to reference numeral 14 is the area of the top surface 6 which is to be in contact with a sample. Preferably the width of each flow path 3 is large, not particularly limited to this, so that fluids such as water, sweat and saliva or other fluids discharged from the sample (hereinafter referred to as fluid different from the sample) are easy to flow.

As the light source 1, any light sources can be used as long as they radiate light having wavelengths absorbed by specific components as objects to be measured. Such light sources include, for example, Globar light source which uses sintered silicon carbide SiC in the form of a rod, CO₂ laser and tungsten lamp. When determining the concentration of, for example, glucose whose absorption peaks appear in the infrared region of wave number 1033 and 1080 cm⁻¹, it is preferable to use a Globar light source from the viewpoint of its capability of covering relatively wide wavelength range and of emitting light satisfactory even in the long wavelength range of about 10 microns.

The light source 1 is disposed so that the light having entered the ATR device 2 through its light-entering surface 11 reaches the water repellent provided portion 7 and held by appropriate means.

The light detection means 5 is arranged in such a position that it receives the light output from the light outputting surface 12 of the ATR device 2. As light detection means 5, those known in the art can be used without special limitation. Such light detection means include, for example, a pyroelectric sensor and a MCT detector.

In the sample contacting area 14, the portion light enters must be limited to the water repellent provided portion 7. In other words, in the sample contacting area 14, the size and position of the water repellent provided portion 7, or the angle of the light-entering surface 11 and the disposition of the light source 1 are determined so that light does not pass through the hydrophilicity provided portion 13.

Then the operation of the concentration measuring apparatus of measuring specific components according to this embodiment will be described with reference to FIG. 1. The light having been radiated from the light source . 1 and entered the light-entering surface 11 of the ATR device 2 propagates within the ATR device 2 while repeating its total reflection. At this time, though the light undergoes total reflection at the interface of the ATR device 2, a slight amount of light oozes outside the ATR device 2. This light is referred to as evanescent wave and known to ooze out to a distance about several times as long as its wavelength. The evanescent wave having oozed into a sample, which is disposed to cover the water repellent provided portion 7, is absorbed in amount corresponding to the constituent components of the sample. The light part of which has been absorbed into the sample is output from the light outputting surface 12 and reaches the light detection means 5. The amount of the light absorbed into the sample is detected by the light detection means 5. And the concentration information of specific components in the sample is calculated based on the amount of the absorbed light detected by the light detection means 5.

When the sample is a lip, the lip is made to cover the sample contacting area 14. In the sample contacting area 14, the size of the water repellent provided portion 7 is determined so that it can be covered with any persons' lip. When a person covers the sample contacting area 14 with his or her lip, his or her saliva intervenes between the lip and the sample contacting area 14. The intervening saliva on the water repellent provided portion 7 is moved toward the hydrophilicity provided portion 13 due to the water repellent action of the water repellent provided portion 7 and the pressure by the lip.

The saliva having been moved to the hydrophilicity provided portion 13 moves through the flow paths 3 to the side surfaces 16 of the ATR device 2 and then moves downward along the side surfaces due to gravity. At this time, the saliva having moved to the flow paths 3 moves to the side surfaces 16 in such a manner as to slide down through the flow paths since hydrophilicity is provided on the inclined plane 4 within the flow paths 3, and falls downward from the ATR device 2. Thus, the saliva supplied from the lip is removed from the water repellent provided portion 7 one after another.

As a result, the light having entered the water repellent provided portion 7 can reach the light detection means 5 while it is substantially unaffected by the saliva, but affected just by the object to be measured (e.g. glucose) in the lip.

Thus, according to the concentration measuring apparatus of this embodiment, the concentration information of specific components in a sample can be determined stably and easily even when fluids different from the sample of organism etc. intervene between the sample and the ATR device 2, since the fluids different from the sample are hard to attach to the water repellent provided portion 7 of the ATR device 2 and layers of the fluids are hard to form between the sample and the ATR device 2.

### [Examples]

In the following, concrete examples of measurements using the concentration measuring apparatus of the embodiment will be shown.

As a light source 1, a Globar light source of sintered silicon carbide SiC in the form of a rod was used. As an ATR device 2 was used a germanium ATR device which is transparent to light of wavelength 1.1 to 10 microns. As light detection means 5, a pyroelectric sensor was used. The water repellent provided portion 7 was formed in such a manner as to coat the top surface 6 of the ATR device 2 with fluorine resin. The hydrophilicity provided portion 13 and the hydrophilicity in the inside of the flow paths 3 were formed and provided in such a manner as to coat the portion of the top surface 6 other than the water repellent provided portion 7 and the inside the flow paths 3 with a solution of lecithin in an organic solvent and dry the same. Providing water repellent and hydrophilicity in the above-described manner allowed the contact angles of the water repellent provided portion 7 and the hydrophilicity provided portion 13 to be about 88 degrees and about 12 degrees, respectively, in contrast with about 44 degrees for germanium.

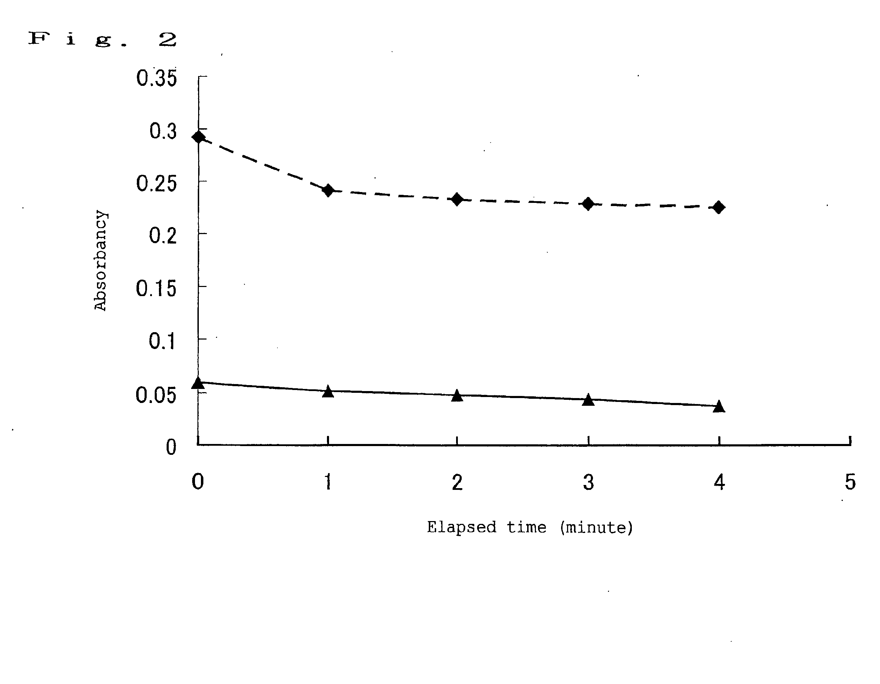

The measured results of absorbance obtained using the concentration measuring apparatuses of this example and a comparative example are shown in FIG. 2. As a comparative example, was used the same concentration measuring apparatus of measuring specific components as this embodiment, except that its ATR device 2 was not provided with the water repellent provided portion 7, the hydrophilicity provided portion 13 and the flow paths 3. As a sample, silicon rubber with water attached thereto was located in such a manner as to cover the water repellent provided portion 7.

In FIG. 2, absorbance is plotted in vertical axis and time course in horizontal axis. The measurement of absorbance was performed at wave number 3200 cm⁻¹, which was the absorption band of water, to measure the effects of water. The solid line in the figure shows the measured results obtained using the concentration measuring apparatus of measuring specific components of this embodiment, whereas the dotted line shows the measured results obtained using the concentration measuring apparatus of measuring specific components of the comparative example.

The graph shows that when using the apparatus of the comparative example, absorbance was large and water had a large effect on the measurement. On the other hand, when using the apparatus of this embodiment, absorbance was small and the effect of water on the measurement was decreased. Presumably, in the use of the apparatus of the comparative example, the measurement was affected largely by water, because water intervened between the sample and the ATR device 2 and the amount of light reaching the sample was decreased, whereas in the use of the apparatus of this embodiment, since the water intervening between the ATR device 2 and the sample was repelled due to water repellent of the water repellent provided portion 7, slid down through the flow paths 3 provided with hydrophilicity and removed, the effect of water on the measurement was decreased.

Thus, it was found that the concentration measuring apparatus of measuring specific components of this invention enables stable and easy determination of concentration information of specific components in a sample even when fluids intervene between its ATR device 2 and the sample (for example, even when the sample is a lip which is apt to affect the measurement due to saliva attaching thereto).

In this example, although silicon rubber with water attached thereto was used as a sample, the same effect can be produced when using any other samples such as organisms' tissues to or from which fluids may attach or exude.

Although the flow paths 3 are connected to part of the hydrophilicity provided portion 13 in the description of the invention so far, the flow paths 3 may be formed in such a manner as to be connected to the water repellent provided portion 7. Even in that case, the same effect as described above can be produced.

Although the number of the flow path 3 is four in the description of the invention so far, the number is not limited to this and flow paths to any number may be formed. But it is preferable to form a larger number of flow paths.

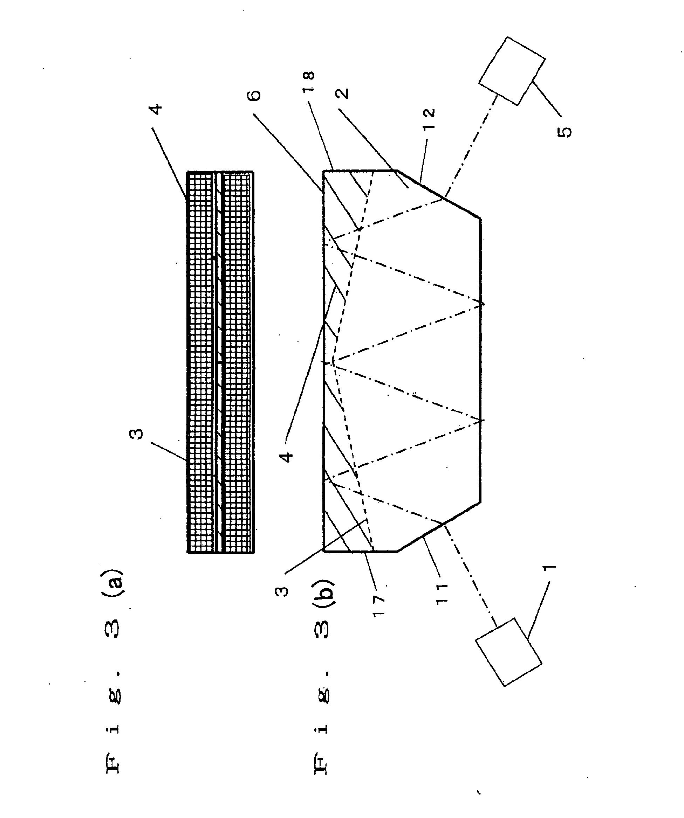

Further, although each flow path 3 is formed with its one opening connected to a side surface 16 in the description of the invention so far, it may be formed with its one opening connected to a surface, other than the side surfaces 16, adjacent to the top surface 6. Such an example is shown in FIGS. 3 (a) and 3 (b) . FIG. 3 shows one example of the apparatuses in which a flow path 3 is formed along the length of the ATR device 2 (in other words, along the optical path through which light having entered the ATR device 2 travels). FIG. 3(a) is a top view and FIG. 3(b) is a side view. In the ATR device 2 shown in FIG. 3, its light-entering surface 11 is not adjacent to its top surface 6, but to its surface 17 adjacent to the top surface 6. And its light-outputting surface 12 is not adjacent to the top surface 6, but to its surface 18 adjacent to the top surface 6. The flow path 3 is formed in such a manner as to be inclined from the neighborhood of the middle of the top surface 6 toward the surfaces 17 and 18 and connected to the surfaces 17 and 18. In such a construction, it is preferable that both light-entering surface 11 and light outputting surface 12 are provided with water repellent.
Alternatively, the construction may be such that the flow path 3 is not connected to the surfaces 17 and 18, but to the side surfaces 16.

Although each flow path 3 is formed with its one opening connected to the side surface 16 in the description of the invention so far, one of the openings of each flow path 3 need not be connected to the side surface 16. In other words, a concave portion may be formed instead of the flow paths 3. In that case, fluids different from a sample do not slide down along the side surfaces 16 through the flow paths 3 ; however, if the concave portion is sufficiently large, the fluids different from the sample can be satisfactorily removed from the water repellent provided portion 7. In that case, the entire hydrophilicity provided portion 13 may be the concave portion. Alternatively, the concave portion may be formed not only in the hydrophilicity provided portion 13, but also in a part of the water repellent provided portion 7, or it may formed in a part of the water repellent provided portion 7 instead of the hydrophilicity provided portion 13. In such cases, the same effect as described above can be produced.

Although hydrophilicity is provided on the inclined plane 4 of each flow path 3 in the description of the invention so far, even when not only the inclined plane but also part of or all over the inside of each flow path is provided with hydrophilicity, the same effect as described above can be produced. Alternatively, even if the inside of each flow path 3 is not provided with hydrophilicity, when the inclination of the inclined plane 4 relative to the top surface 6 is sufficiently large and the width of each flow path 3 is also sufficiently large, fluids different from a sample slide down along the side surfaces 16, and therefore, the same effect as described above can be produced.

Although the water repellent provided portion 7 is formed in the middle of the top surface 6 of the ATR device 2 in the description of the invention so far, it may be formed in any other area as long as, to say the least of it, the area is in contact with a sample and the light for irradiating the sample enters the area. For example, the water repellent provided portion 7 may be formed in the entire sample contacting area 14 or on the entire top surface 6 shown in FIG. 1.

When the water repellent provided portion 7 is formed on the entire top surface 6, the hydrophilicity provided portion 13 may or need not be formed on the side surfaces 16. When the hydrophilicity provided portion 13 is formed on the side surfaces 16, fluids different from the sample is removed more quickly from the water repellent provided portion 7. However, even if the hydrophilicity provided portion 13 is not formed on the side surfaces 16, once the fluids different from the sample which have been repelled from the water repellent provided portion 7 reach the side surfaces 16, they fall down along the side surfaces 16 by the action of gravity; accordingly, the same effect as described above can be produced. When the side surfaces 16 (in other words, the surfaces which are adjacent to the top surface 6 and not on the optical path) are provided with hydrophilicity, hydrophilicity may be provided for a part of each side surface 16 or the entire part of each side surface 16 . Increase in area of hydrophilic surfaces increases the area of the surface capable of adsorbing the fluids different from the sample. As a result, the effect of the fluids different from the sample can be decreased, which enables stable and easy determination of concentration information of specific components in the sample.

When the side surfaces 16 are not provided with hydrophilicity, at least part of the side surfaces may be provided with water repellent. If the side surfaces 16 are provided with water repellent, the fluids different from the sample, which is on the water repellent provided portion 7 of the top face 6, can be removed quickly, because once the fluids have been introduced to the side surfaces, they can slide down the side surfaces not only by the action of gravity, but also by the water repellent provided for the side surfaces.

Although the water repellent provided portion 7 is formed on part of the ATR device 2 and the hydrophilicity provided portion 13 is formed in the area adjacent to the water repellent provided portion 7 in the description of the invention so far, once the water repellent provided portion 7 is formed by providing a predetermined part of the ATR device 2 with water repellent, in the area adjacent to the water repellent provided portion 7, which is to be the hydrophilicity provided portion 13, the contact angle of the ATR device 2 itself can be utilized. Even in such a case, the same effect as described above can be produced.

Alternatively, once the area of the ATR device 2 other than the area to be the water repellent provided portion 7 is processed to provide the same with hydrophilicity, in the area to be the water repellent provided portion 7, the contact angle of the ATR device 2 itself can be utilized. Even in such a case, the same effect as described above can be produced.

Although the invention has been described, on the assumption that the area of the water repellent provided portion 7 is water repellent and the area of the hydrophilicity provided portion 13 is hydrophilic, both the water repellent provided portion 7 and the hydrophilicity provided portion 13 may be water repellent or hydrophilic. Even in such a case, the same effect as described above can be produced, if the contact angle of the water repellent provided portion 7 is larger than that of the hydrophilicity provided portion 13.

In the above-described example, for example, a germanium ATR device 2 was used, but an ATR device of other materials may also be used. For example, the contact angle of silicon is about 90 degrees and that of ZnSe is about 81 degrees, and once the material for the ATR device 2 is selected, the area whose contact angle is larger than that of the material for the ATR device 2 and the area whose contact angle is smaller than that of the same can be formed depending on the material for the ATR device 2.

Although the water repellent provided portion 7 and the hydrophilicity provided portion 13 are formed on the ATR device 2 in the description of the invention so far, it is also possible to form the flow paths 3 alone in such a manner as to allow them to be in communication with the sample contacting area 14 without forming neither the water repellent provided portion 7 nor the hydrophilicity provided portion 13. Even in such a case, the same effect as described above can be produced.

As specific components, any substances can be selected as long as they are optically measurable. Such components include, for example, blood sugar level, water, cholesterol, neutral fat, lactic acid, blood ethanol and various components of body fluid in organisms.

As a sample, any samples can be used as long as they contain optically measurable substances. Such samples include, for example, tissues of organisms such as skin and lip.

The concentration information includes, for example, absolute value of the concentration of specific components, component ratio and composition, and changes thereof with time.

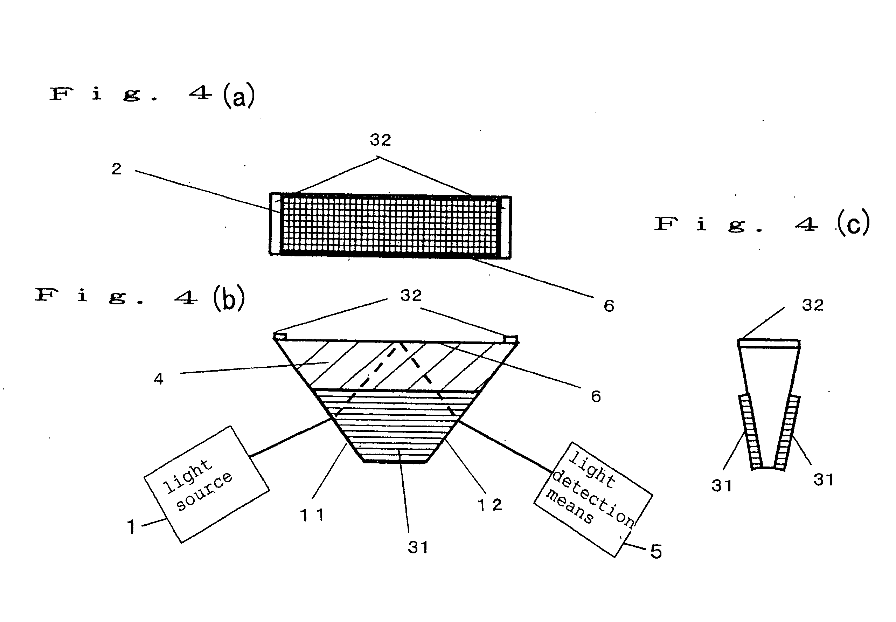

In the concentration measuring apparatus of this embodiment, may be arranged steps 32, as an example of fluid inflow preventive means of the present invention, which prevent fluids different from the sample from flowing in the light-entering surface 11 or the light outputting surface 12 from the top surface 6 of the ATR device 2. The construction of the concentration measuring apparatus including steps 32 is shown in FIG. 4. FIG. 4 (a) is a top view of the ATR device 2 when the concentration measuring apparatus include steps 32, FIG. 4(b) is a side view of the apparatus, and FIG. 4(c) a view of the ATR device 2 seen from light-entering surface 11 side or the light outputting surface 12 side . The steps 32 are formed at, for example, such positions that the light-entering surface 11 and the top surface 6 are in contact with each other on the top surface 6 and the light outputting surface 12 and the top surface 6 are in contact with each other on the top surface 6 in such a manner as to be sufficiently high to prevent the fluids different from the sample from flowing in the light-entering surface 11 or the light outputting surface 12.

Although an example is shown in FIG. 4 in which steps 32 are arranged as fluid inflow preventive means of the present invention, the fluid inflow preventive means of the present invention may be a concave portion which is for holding fluids. Alternatively, the fluid inflow preventive means may be flow paths for letting fluids go from the top surface 6 of the ATR device 2 toward the side surfaces 16.

As fluid inflow preventive means, may be provided suction means 31 for sucking fluids different from the sample. It is conceivable that sponges as suction means are stuck on the side surfaces 16, as shown in FIG. 4 (b), so that fluids different from the sample do not enter the light-entering surface 11 or the light outputting surface 12 from the side surfaces 16.

Alternatively, the above-described steps 32 may be formed of sponge, or a sponge may be inserted into the concave portion, as fluid inflow preventive means. Further, the construction may be such that a sponge is inserted into each flow path, as fluid inflow preventive means, and part of the sponge is connected to each side surface 16. The suction means 31 may be not only a sponge but also an absorber such as paper, or may be means, such as vacuum pump, which sucks fluids using vacuum. In addition, means known in the art can be used without any limitation.

Although the optical device of the present invention is an ATR device 2 in the description of the invention so far, it is not limited to the ATR device 2, and any types of optical devices may also be used. Such devices include, for example, devices for measuring scattering light scattering in a sample and devices for measuring transmitted light having been transmitted through a sample. Even in such cases, if the water repellent provided portion 7 is formed in the portion where the optical device is in contact with the sample and the hydrophilicity provided portion 13 is formed around the water repellent provided portion 7, the same effect as described above can be produced.

Although the concentrations of specific components are determined by the amount of the light returned from the sample which the light detection means 5 absorbs in the description of the invention so far, it is also conceivable that the light detection means 5 includes spectroscopic means. In such a case, the wavelength dependency of the specific components in the sample can be measured. It is also conceivable that the light detection means 5 includes an interferometer with which FT-IR is measured. In such a case, measurement of high sensitivity is made possible.

Although the concentrations of specific components in a sample are measured by receiving light returned from the sample and depending on the received light in the description of the invention so far, the object to be measured is not limited to concentration, information other than concentration, such as temperature, humidity, viscosity and density, may also be measured. Even in such cases, if the information corresponds to the received light, the same effect as described above can be produced.

### Industrial Application

According to the present invention, an information measuring apparatus can be provided which enables stable and easy determination of information such as concentration of specific components in a sample even when fluids, such as water, saliva and sweat, intervene between its optical device and the sample.

## Claims

1. An information measuring apparatus of measuring information in a sample, comprising: a light source; an optical device which irradiates light input from the light source to the sample in contact with the optical device, receives the light returned from the sample, and outputs the received light; and light detection means of detecting the amount of the light output from the optical device,
the optical device having a first area that is a part of the face in contact with the sample and to which the light for irradiating the sample inputs and a second area adjacent to the first area, and the contact angle in the first area being larger than that in the second area,
wherein the information in the sample is measured based on the signal obtained through the light detection means.

2. The information measuring apparatus according to claim 1, wherein the information is the concentration information of specific components in the sample.

3. The information measuring apparatus according to claim 1, wherein the first area is formed of the material which the optical device is formed of.

4. The information measuring apparatus according to claim 1, wherein the second area is formed of the material which the optical device is formed of.

5. The information measuring apparatus according to claim 1, wherein an concave portion is formed in part of the first area or at least in part of the second area.

6. The information measuring apparatus according to claim 1 or 3, wherein the first area is water repellent.

7. The information measuring apparatus according to claim 1 or 4, wherein the second area is hydrophilic.

8. The information measuring apparatus according to claim 1, wherein the first area is water repellent and the second area is hydrophilic.

9. The information measuring apparatus according to claim 1, wherein the optical device is an attenuation total reflection device

10. The information measuring apparatus according to claim 5, wherein the concave portion is connected to the surface which is in contact with the sample and not on the optical path of the light passing through the optical device and flow paths are formed for discharging fluids intervening between the first area and the sample.

11. The information measuring apparatus according to claim 10, wherein at least part of the flow paths is hydrophilic.

12. The information measuring apparatus according to claim 10, wherein the flow paths are formed so that they open in part of the first area.

13. The information measuring apparatus according to claim 5, wherein the concave portion is formed in such a position and in such a size that it does not substantially prevent the optical path of the light passing through the optical device.

14. The information measuring apparatus according to claim 1, wherein at least part of the surfaces of the optical device, which are adjacent to the surface in contact with the sample and are not on the optical path of the light passing through the optical device, is hydrophilic.

15. The information measuring apparatus according to claim 1, wherein at least one of the surfaces of the optical device which are adjacent to the surface in contact with the sample is water repellent.

16. The information measuring apparatus according to claim 1, further comprising fluid inflow preventive means of preventing the fluids intervening between the first area and the sample from flowing in the surface of the optical device through which the light from the light source enters and the surface of the optical device through which the light returned from the sample is output into the light detection means from the first or the second area of the optical device.

17. The information measuring apparatus according to claim 1, further comprising suction means of sucking the fluids, which is provided on the surface of the optical device adjacent to the surface in contact with the sample.
